# EUROPÄISCHE PATENTANMELDUNG

(11) **EP 0 564 919 A1**
(43) Veröffentlichungstag der Anmeldung: **13.10.1993**
(21) Anmeldenummer: 93104984.5
(22) Anmeldetag: 25.03.1993
(51) Int. Cl.: C07C 67/343, C07C 69/734

(54) **Verfahren zur Herstellung von Zimtsäurederivaten**

(30) Priorität: 07.04.1992 DE 4211608
(71) Anmelder: BAYER AG, 51368 Leverkusen (DE)
(72) Erfinder: Kaufmann, Dieter, W-5060 Bergisch Gladbach 2 (DE); Hesse, Carsten, Dr., W-5090 Leverkusen (DE); Himmler, Thomas, Dr., W-5068 Odenthal (DE)

(57) **Zusammenfassung**

Zimtsäurederivate, insbesondere p-Methoxyzimtsäure-2-ethylhexylester und p-Methoxyzimtsäure-2-isoamylester, werden unter Vermeidung von besonderem Aufwand für die Arbeitsplatzhygiene und Ökologie hergestellt, indem man Bromaromaten und Acrylsäurederivate in Gegenwart von Palladium-Katalysatoren, einem großen Überschuß von Phosphan (bezogen auf Palladium), in Gegenwart einer anorganischen Base und in Gegenwart von Alkoholen und/oder von einem Phasertransfer-Katalysator arbeitet.

## Beschreibung

Die vorliegende Erfindung betrifft ein Verfahren zur Herstellung von Zimtsäurederivaten, bei dem hinsichtlich Lösungsmitteln und Basen kein besonderer Aufwand erforderlich ist.

Es ist bereits bekannt, daß Olefine in Gegenwart von Palladium-Katalysatoren, Phosphanliganden und einer Base durch Halogenaromaten aryliert werden können (siehe R.F. Heck, Org. React. 27, 345 - 391 (1982)).

Die Reaktion wird häufig mit verschiedenen Aminen (z.B. Triethylamin, Tributylamin, Tetramethylethylendiamin) durchgeführt, die gleichzeitig als Lösungsmittel dienen können. Als Basen können auch Alkalicarboxylate in Dimethylformamid (siehe EP-OS 78 768) oder Alkalicarbonate oder -hydrogencarbonate eingesetzt werden, wenn gleichzeitig ein polares aprotisches Lösungsmittel (z.B. Dimethylformamid, Hexamethylphosphorsäuretriamid, Acetonitril) und ein Phasentransferkatalysator zugegen sind (siehe Tetrahedron Lett. 26, 2667-2670 (1985) und J. Org. Chem. 56, 1289-1293 (1991)).

Alle diese Base/Lösungsmittel-Systeme stellen aggressive Medien dar und erfordern aus toxikologischer und ökologischer Sicht heraus besonderen Aufwand für ihre Handhabung.

Aus der Patentanmeldung WO 90/10617 ist außerdem bekannt, daß man p-Methoxyanilin (=p-Anisidin) in einem zweistufigen Prozeß zunächst diazotieren und dann mit Kaliumiodid in 4-Iodanisol überführen kann. Dieses kann danach in Gegenwart von Triethylamin und einem Palladium-Katalysator mit Acrylsäure-2-ethylhexylester zum 4-Methoxyzimtsäure-2-ethylhexylester umgesetzt werden. Auch hier wird das im Prinzip unerwünschte Triethylamin benötigt und das sich bildende Triethylammoniumiodid muß mit Alkalilauge wieder in Triethylamin und Alkaliiodid gespalten werden. Dieses Verfahren ist sehr vielstufig und erfordert die Rückgewinnung von Alkaliiodid.

Die Reaktion von 4-Iodanisol mit Acrylsäuremethylester und einer stöchiometrischen Menge Tributylamin in Gegenwart von 1 mol-% Palladium(II)-acetat ergibt nach 5 Stunden bei 100°C nur eine Ausbeute von 68 % an 4-Methoxyzimtsäuremethylester (J. Org. Chem. 37, 2320-2322 (1972)).

Die Umsetzung von 4-Bromanisol mit Acrylsäuremethylester und einer stöchiometrischen Menge Tetramethylethylendiamin in Gegenwart von 1 bis 2 mol-% Palladium(II)-acetat und 2 bis 4 mol-% Triphenylphosphan führt nach 36 Stunden bei 135°C zu einer Ausbeute an 4-Methoxyzimtsäuremethylester von 54 % (J. Am. Chem. Soc. 96, 1133-1136 (1974)).

Es besteht deshalb das Bedürfnis nach einem Verfahren, mit dem die weniger reaktiven, aber leichter herzustellenden Bromaromaten in Gegenwart von Palladiumkatalysatoren mit Acrylsäurederivaten in hohen Raum-Zeit-Ausbeuten und unter Verwendung von keinen besonderem Aufwand erfordernden Hilfsmitteln umgesetzt werden können.

Es wurde nun ein Verfahren zur Herstellung von Zimtsäure-Derivaten aus Bromaromaten und Acrylsäurederivaten in Gegenwart von Palladiumkatalysatoren und einem Phosphan gefunden, das dadurch gekennzeichnet ist, daß man in Gegenwart von einer anorganischen Base von Phosphan im großen Überschuß (bezogen auf Palladium) und von Alkohol und/oder von einem Phasen-Transfer-Katalysator arbeitet.

Bei diesem Verfahren wird kein Amin und/oder Lösungsmittel benötigt, dessen Handhabung besonderen Aufwand erfordert.

Das Palladium kann in das erfindungsgemäße Verfahren in beliebiger Form zugegeben werden. Bevorzugt sind Palladium-Verbindungen der Oxidationsstufen 0 und +2, beispielsweise Palladium(II)-halogenide, Palladium(II)-carboxylate, und komplexe Palladium-Verbindungen, beispielsweise komplexe Palladium-Verbindungen der Typen PdX₂L₂ und PdL₄, wobei X für ein Halogenatom und L für einen Liganden (beispielsweise für Triphenylphosphan, Tri(o-tolyl)phosphan oder Tri(p-anisyl)phosphan steht. Die Menge des Palladiums kann so gewählt werden, daß, bezogen auf den eingesetzten Bromaromaten, 0,0001 bis 1 mol-% Palladium im Reaktionsgemisch vorliegen. Vorzugsweise beträgt diese Menge 0,001 bis 0,1 mol-%, besonders bevorzugt 0,005 bis 0,05 mol-%.

Als Acrylsäurederivate können beispielsweise solche der Formel (I) eingesetzt werden 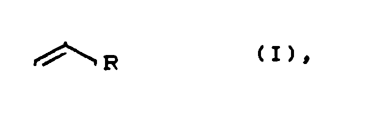
in der
- R: für CN oder COR¹ steht, wobei R¹ OH, O-C₆-C₁₀-Aryl, O-C₁-C₂₀-Alkyl, NH₂, NH-C₆-C₁₀-Aryl, NH-C₁-C₂₀-Alkyl, N-C₆-C₁₀-Aryl, N-C₁-C₂₀-Alkyl-C₆-C₁₀-Aryl oder N-di-C₁-C₂₀-Alkyl bedeutet.

In Formel (I) steht R vorzugsweise für COR¹ mit R¹ = O-C₁-C₂₀-Alkyl, besonders bevorzugt C₁-C₁₀-Alkyl.

Als Bromaromaten können beispielsweise Brombenzole der Formel (II) eingesetzt werden 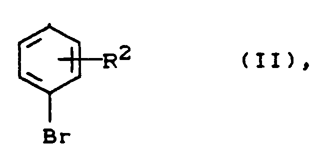
in der
- R²: für Wasserstoff, C₆-C₁₀-Aryl, C₁-C₂₀-Alkyl, OR³ oder NR³₂ mit R³ Wasserstoff, C₆-C₁₀-Aryl oder C₁-C₂₀-Alkyl steht.

In Formel (II) steht R² vorzugsweise für OR³ oder NR³₂ mit R³ = Phenyl oder C₁-C₁₀-Alkyl, besonders bevorzugt steht R² für Methoxy.

Das Molverhältnis von Bromaromat zu Acrylsäurederivat kann beliebig gewählt werden. Vorzugsweise arbeitet man bei einem Molverhältnis von Bromaromat zu Acrylsäurederivat im Bereich von 1:0,7 bis 1:3. Besonders bevorzugt beträgt dieses Verhältnis 1:0,8 bis 1:1,2.

Für das erfindungsgemäße Verfahren kommen als anorganische Basen beispielsweise Alkali- und Erdalkalisalze schwacher Säuren, vorzugsweise Alkali- und Erdalkalihydrogencarbonate und/oder -carbonate in Frage. Besonders bevorzugt wird Natriumcarbonat verwendet. Das Verhältnis von Bromaromat zu Base wird vorzugsweise so gewählt, daß pro Mol Bromaromat 0,8 bis 2, besonders bevorzugt 0,9 bis 1,3 Äquivalente Base eingesetzt werden.

Sofern das erfindungsgemäße Verfahren in Gegenwart eines Phasen-Transfer-Katalysators durchgeführt wird, kommen an sich bekannte Phasen-Transfer-Katalysatoren infrage. Es können im Rahmen der vorliegenden Erfindung beispielsweise Kronenether, jeweils mit organischen Resten substituierte Ammoniumsalze oder Phosphoniumsalze und Polyethylenglykole als Phasentransferkatalysatoren eingesetzt werden. Bevorzugt sind Ammonium-und Phosphoniumsalze, die als organische Reste C₆-C₁₀-Aryl-, C₇-C₁₂-Aralkyl-und/oder C₁-C₂₀-Alkyl-Reste und als Anion ein Halogenidion, insbesondere Chlorid, enthalten, sowie Polyethylenglykole mit einem mittleren Molekulargewicht von 200 bis 1000 g/mol. Besonders bevorzugt werden Methyltrioctylammoniumchlorid und Polyethylenglykol 400 als Phasen-Transfer-Katalysator eingesetzt. Die Menge des Phasen-Transfer-Katalysators kann beispielsweise 0,01 bis 50 mol-%, bezogen auf eingesetzten Bromaromaten, betragen. Vorzugsweise beträgt diese Menge 0,1 bis 15 mol-%, besonders bevorzugt 0,7 bis 2 mol-%.

Sofern das erfindungsgemäße Verfahren in Gegenwart von Alkoholen durchgeführt wird können dafür beispielsweise C₂-C₂₄-Alkohole eingesetzt werden. Bevorzugt sind C₄-C₂₀-Alkohole, ganz besonders bevorzugt C₅-C₁₈-Alkohole. Unter der Bezeichnung "Alkohole" werden hier Mono- und Polyole der entsprechenden C-Zahl verstanden. Als Beispiele seien genannt: 3-Methyl-1-butanol, 2-Ethyl-1-hexanol, 1-Decanol, 1-Dodecanol, 1-Octadecanol, 1,12-Octadecandiol, 2-Ethyl-1,3-hexandiol, 1,2,3-Propantriol und 2,2-Bis-(hydroxymethyl)-1,3-propandiol genannt. Natürlich können auch Mischungen solcher Alkohole verwendet werden. Die Menge an Alkoholen kann in weiten Grenzen variiert werden. Bevorzugt beträgt sie 0,1 bis 2, besonders bevorzugt 0,2 bis 1 Liter Alkohol pro Mol Bromaromat.

Es ist auch möglich, beides, einen Phasentransfer-Katalysator und Alkohole gemeinsam einzusetzen. Dabei kann die Menge an Alkohol deutlich kleiner gewählt werden. In diesem Fall ist es im allgemeinen ausreichend 1 bis 50 mol-%, bezogen auf eingesetzten Bromaromaten, bevorzugt 3 bis 30 mol-% Alkohol zuzugeben.

Als Phosphan kann beispielsweise ein Phosphan der Formel (III) eingesetzt werden 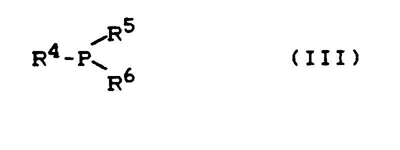
in der
- R⁴, R⁵, R⁶: unabhängig voneinander jeweils für gegebenenfalls durch Halogen, C₁-C₂₀-Alkyl, OR⁷ oder NR⁷₂ (R⁷ = Wasserstoff oder C₁-C₂₀-Alkyl), substituiertes C₆-C₁₀-Aryl, C₁-C₂₀-Alkyl, Phosphino-C₁-C₂₀-Alkyl oder Phosphino-C₆-C₁₀-Aryl
stehen.

Vorzugsweise stehen in Formel (III) R⁴, R⁵ und R⁶ unabhängig voneinander für durch C₁-C₂₀-Alkyl und/oder C₁-C₂₀-Alkoxy substituierte und/oder unsubstituierte C₆-C₁₀-Arylreste.

Besonders bevorzugte Phosphane der Formel (III) sind Triphenylphosphan, Tri-o-tolylphosphan, Tri-p-anisylphosphan und Bisdiphenylphosphinomethan.

Phosphane können in das erfindungsgemäße Verfahren beispielsweise in einem Mol-Verhältnis von Palladium:Phosphor von 1:4 bis 1:150 eingesetzt werden. Bevorzugt beträgt dieses Verhältnis 1:10 bis 1:100, besonders bevorzugt 1:20 bis 1:90.

Das erfindungsgemäße Verfahren kann man beispielsweise bei Temperaturen im Bereich von 100 bis 300°C durchführen. Bevorzugte Temperaturen liegen im Bereich von 120 bis 250°C, besonders bevorzugte im Bereich von 140 bis 190°C. Das erfindungsgemäße Verfahren wird üblicherweise bei Normaldruck durchgeführt. Es kann jedoch auch bei vermindertem oder erhöhtem Druck durchgeführt werden. Die Anwendung von erhöhtem Druck ist insbesondere dann angezeigt, wenn man bei einer Reaktionstemperatur arbeiten möchte, bei der einzelne Bestandteile des Reaktionsgemisches bei Normaldruck sieden.

Falls in das erfindungsgemäße Verfahren höhere Alkohole eingesetzt werden, die mit Wasser nicht unbegrenzt mischbar sind, ist es im allgemeinen vorteilhaft, bei der Rückflußtemperatur des Reaktionsgemisches zu arbeiten und sich bildendes Reaktionswasser kontinuierlich abzutrennen, z.B. mit Hilfe eines Wasserabscheiders.

Das erfindungsgemäße Verfahren wird im allgemeinen unter einem Schutzgas, z.B. Stickstoff, und unter Rühren durchgeführt.

Mit dem erfindungsgemäßen Verfahren können beispielsweise Zimtsäurederivate der Formel (IV) hergestellt werden 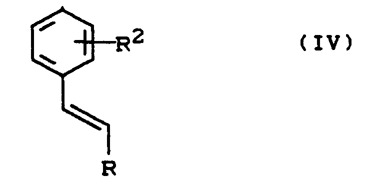
in der die verwendeten Symbole die bei den Formeln (I) und (II) angegebene Bedeutung haben.

Nach Durchführung des erfindungsgemäßen Verfahrens können die entstandenen anorganischen Salze beispielsweise durch einfaches Abfiltrieren oder Abnutschen abgetrennt werden. Man kann die Salze auch absitzen lassen und die restliche Reaktionsmischung abdekantieren.

Eine mögliche Ausführungsform des erfindungsgemäßen Verfahrens wird im folgenden beispielhaft an der Umsetzung von 4-Bromanisol mit Acrylsäure-2-ethylhexylester erläutert:
4-Bromanisol, Acrylsäure-2-ethylhexylester, Natriumcarbonat und Methyl-tri-n-octylammoniumchlorid werden vorgelegt. Triphenylphosphan und Palladium(II)chlorid werden in einem Mol-Verhältnis von Palladium:Phosphor von 1:40 zugesetzt. Die Reihenfolge der Zugabe dieser Komponenten kann beliebig verändert werden. Dann wird das Gemisch unter Stickstoff und kräftigem Rühren auf 150 bis 160°C erhitzt. Wenn festgestellt worden ist, daß sich kein 4-Bromanisol mehr umsetzt, wird die Reaktion abgebrochen,
das heißt, auf Raumtemperatur abgekühlt. Dann wird ausgefallenes Natriumbromid abgesaugt und der Filterkuchen mit einem organischen Lösungsmittel (z.B. Petrolether) gewaschen, um anhaftendes Produkt zu entfernen. Das erhaltene Filtrat kann vom Lösungsmittel befreit und im Vakuum destilliert werden.

Es ist überraschend, daß die erfindungsgemäße palladiumkatalysierte Umsetzung von weniger reaktiven Bromaromaten mit Acrylsäurederivaten in Gegenwart ungelöst vorliegender anorganischer Basen bei Zusatz eines Phasentransfer-Katalysators und/oder Alkoholen auch bei Einsatz nur sehr kleiner Mengen Palladiumkatalysator hohe Raum-Zeit-Ausbeuten ergibt.

Ebenfalls überraschend ist, daß der Zusatz von Phosphan in großem Überschuß die Reaktionsgeschwindigkeit deutlich erhöht. Es ist bekannt, daß Palladium-Komplexe, die die Arylierung von Olefinen katalysieren, mit höchstens 4 Phosphan-Liganden umgeben sind, so daß ein optimales Verhältnis von Palladium:Phosphor von 1:4 zu erwarten war. Da bei geringen Konzentrationen an Palladium üblicherweise mit einem geringen Überschuss an Phosphan gearbeitet wird, damit in jedem Fall die Bildung der 1:4-Komplexe gewährleistet ist, wäre es nicht überraschend gewesen, bei der Steigerung des Verhältnisses von Palladium:Phosphor auf beispielsweise 1:8 Vorteile zu erhalten. Die weitere Steigerung des Überschusses an Phosphanen auf Mol-Verhältnisse von Palladium:Phosphan von über 1:10 und die damit verbundene Erhöhung der Reaktionsgeschwindigkeit war jedoch nicht zu vorauszusehen, insbesondere da bei anderen Reaktionsbedingungen ein Molverhältnis Palladium:Phosphan von 1:2 als optimal bezeichnet wird (J. Am. Chem. Soc. 96, 1133-1136 (1974)) und beim Einsatz von Palladium:Phosphan im Molverhältnis 1:8 keine nennenswerten Änderungen gegenüber der Arbeitsweise bei einem Molverhältnis von 1:2 gefunden wurden (J. Organomet. Chem. 258, 101-108 (1983)).

Das erfindungsgemäße Verfahren ermöglicht die Herstellung von Zimtsäurederivaten, insbesondere die Herstellung von p-Methoxyzimtsäure-2-ethylhexylester und 4-Methoxyzimtsäureisoamylester, unter vorteilhaften Reaktionsbedingungen, wobei kein besonderer Aufwand für die Handhabung von Hilfsmitteln (Basen, Lösungsmittel) nötig ist. Die benötigten Basen, Phasen-Transfer-Katalysatoren und Alkohole sind leicht zugänglich und preisgünstig.

Das erfindungsgemäße Verfahren ist wesentlich wirtschaftlicher und erreicht im Vergleich zu den Verfahren des Standes der Technik eine höhere Raum-Zeit-Ausbeute.

Zimtsäurederivate, insbesondere p-Methoxyzimtsäure-2-ethylhexylester und -isoamylester können beispielsweise als UV-Absorber in Kosmetika eingesetzt werden (siehe US-PS 5 008 100 und US-PS 4 810 490).

### Beispiel 1

In einem Vierhalsrundkolben wurden 187,04 g 4-Bromanisol, 202,7 g Acrylsäure-2-ethylhexylester, 53 g Natriumcarbonat und 4,42 g Methyltri-n-octylammoniumchlorid vorgelegt und mit 2,63 g Triphenylphosphan und 56 mg Palladiumacetat (0,025 mol-% bezogen auf 4-Bromanisol) versetzt. Das Gemisch wurde unter Stickstoff und kräftigem Rühren auf 150 bis 160°C erhitzt. Nach 20 Stunden betrug der Umsatz gemäß Gaschromatographie 98 %, bezogen auf eingesetztes 4-Bromanisol.

Das ausgefallene Natriumbromid (93,5 g = 91 % d. Th.) wurde abgesaugt und mit wenig Hexan gewaschen. Das Filtrat wurde vom Hexan befreit und im Vakuum destilliert. Es ergab sich eine Ausbeute an isoliertem p-Methoxyzimtsäure-2-ethylhexylester von 187,1 g.

### Beispiel 2 (Vergleichsbeispiel)

Es wurde verfahren wie in Beispiel 1, jedoch wurden nur 263 mg Triphenylphosphan zugesetzt, so daß ein Mol-Verhältnis von Palladium:Phosphor zu 1:4 resultierte. Nach 20 Stunden betrug der Umsatz gemäß Gaschromatographie 30 %, bezogen auf eingesetztes 4-Bromanisol.

### Beispiel 3

Es wurde verfahren wie in Beispiel 1, jedoch wurde statt Palladiumacetat 44 mg Palladiumchlorid zugesetzt. Nach 20 Stunden betrug der Umsatz gemäß Gaschromatographie 95 %, bezogen auf eingesetztes 4-Bromanisol.

### Beispiel 4

Es wurde verfahren wie in Beispiel 1, jedoch wurde statt Methyltri-n-octylammoniumchlorid 4 g Polyethylenglykol 400 zugesetzt. Nach 4 Stunden betrug der Umsatz gemäß Gaschromatographie 89 %, bezogen auf eingesetztes 4-Bromanisol.

### Beispiel 5

Ein Reaktionsgemisch aus 18,7 g 4-Bromanisol, 20,3 g Acrylsäure-2-ethylhexylester, 6,36 g Natriumcarbonat, 0,0025 g [= 0,01 mol-%] Palladium(II)-acetat und 0,026 g Triphenylphosphan (Molverhältnis Pd:Phosphor=1:10) in 80 ml 2-Ethyl-1-hexanol wurde unter Stickstoff auf 150°C erhitzt. Der gaschromatographisch ermittelte Umsatz an Bromanisol betrug nach 4 Stunden 51 %.

### Beispiel 6

Es wurde vorgegangen wie in Beispiel 5, jedoch wurden 0,105 g Triphenylphosphan eingesetzt (=Molverhältnis Pd:Phosphor 1:40). Der Umsatz betrug nach 4 Stunden 98 %.

### Beispiel 7

Es wurde vorgegangen wie in Beispiel 6, jedoch wurde die Reaktion bei der Rückflußtemperatur des Reaktionsgemisches durchgeführt. Der Umsatz nach 2 Stunden betrug 100 %.

### Beispiel 8

Ein Reaktionsgemisch aus 18,7 g 4-Bromanisol, 20,3 g Acrylsäure-2-ethylhexylester, 6,36 g Natriumcarbonat, 0,056 g (0,025 mol-%) Palladium(II)-acetat und 0,088 g Tri(p-anisyl)phosphan in 80 ml 2-Ethyl-1-hexanol wurde unter Stickstoff auf 180°C erhitzt. Der gaschromatographisch ermittelte Umsatz an Bromanisol betrug nach 2 Stunden 99 %.

### Beispiel 9

Ein Reaktionsgemisch aus 18,7 g 4-Bromanisol, 20,3 g Acrylsäure-2-ethylhexylester, 6,36 g Natriumcarbonat, 0,0175 g (0,025 mol-%) Bis(triphenylphosphan)palladium(II)-chlorid und 0,0525 g Triphenylphosphan (Molverhältnis Pd:Phosphor insgesamt 1:10) in 80 ml 2-Ethyl-1-hexanol wurde unter Stickstoff auf 150°C erhitzt. Der gaschromatographisch ermittelte Umsatz an Bromanisol betrug nach 4 Stunden 99 %.

### Beispiel 10

Ein Reaktionsgemisch aus 18,7 g 4-Bromanisol, 20,3 g Acrylsäure-2-ethylhexylester, 6,36 g Natriumcarbonat, 0,0056 g Palladium(II)-acetat und 0,262 g Triphenylphosphan (Molverhältnis Pd:Phosphor 1:40) in 10 ml 2-Ethyl-1-hexanol wurde unter Stickstoff auf 150°C erhitzt. Der gaschromatographisch ermittelte Umsatz an Bromanisol betrug nach 3 Stunden 53 %.

### Beispiel 11

Es wurde vorgegangen wie in Beispiel 10, jedoch wurden 20 ml 2-Ethyl-1-hexanol eingesetzt. Der Umsatz betrug nach 3 Stunden 91 %.

### Beispiel 12

Es wurde vorgegangen wie in Beispiel 10, jedoch wurden 40 ml 2-Ethyl-1-hexanol eingesetzt. Der Umsatz betrug nach 2 Stunden 86 %, nach 3 Stunden 100 %.

### Beispiel 13

Es wurde vorgegangen wie in Beispiel 10, jedoch wurden 80 ml 2-Ethyl-1-hexanol eingesetzt. Der Umsatz nach 2 Stunden betrug 97 %.

### Beispiel 14

Ein Reaktionsgemisch aus 18,7 g 4-Bromanisol, 15,6 g Acrylsäureamylester, 6,36 g Natriumcarbonat, 0,0056 g (0,025 mol-%) Palladium(II)-acetat, 0,262 g Triphenylphosphan (Molverhältnis Pd:Phosphor 1:40) und 80 ml Isoamylalkohol wurde unter Stickstoff auf Rückflußtemperatur erhitzt. Der Umsatz an 4-Bromanisol betrug nach 6 Stunden 94 %.

### Beispiel 15

167 g 4-Bromanisol, 184,3 g Acrylsäure-2-ethylhexylester, 664 g 2-Ethyl-1-hexanol, 55,7 g Natriumcarbonat und 0,66 g Triphenylphosphan wurden in einem 4 1-Kolben vorgelegt. Nach einigen Minuten Rühren bei Raumtemperatur wurde eine Lösung von 0,056 g Palladium(II)-acetat in 20 g 4-Bromanisol zugegeben. Anschließend wurde das Reaktionsgemisch 7 Stunden unter einer Stickstoffatomosphäre bei Rückflußtemperatur gut gerührt. Nach Abkühlen auf Raumtemperatur wurde das Reaktionsgemisch filtriert und der Filterrückstand mit 150 g 2-Ethyl-1-hexanol nachgewaschen. Der resultierende Filterrückstand wurde im Vakuum getrocknet. Es wurden so 101,7 g Feststoff erhalten (überwiegend Natriumbromid und Natriumcarbonat).

Die fraktionierte Destillation des Filtrates ergab 775,7 g 2-Ethyl-1-hexanol und 237 g 4-Methoxyzimtsäure-2-ethylhexylester entsprechend 81,6 % der Theorie.

### Beispiel 16

171,7 g 4-Bromanisol, 332 g 2-Ethyl-1-hexanol, 63,6 g Natriumcarbonat und 2,1 g Triphenylphosphan (Molverhältnis Pd:Phosphor 1:80) wurden in einem 2 1-Kolben vorgelegt und 15 Minuten unter Stickstoffeinleitung zum Rückfluß erhitzt. Anschließend wurden eine Lösung von 0,022 g Palladium(II)-acetat in 15,3 g 4-Bromanisol und 203 g Acrylsäure-2-ethylhexylester zugegeben. Nach 3 Stunden Rückfluß am Wasserabscheider (zur Abtrennung des bei der Reaktion gebildeten Wassers) betrug der Umsatz an 4-Bromanisol 90 %.

Die Filtration des Reaktionsgemisches und Destillation des Filtrats ergab eine Ausbeute an 4-Methoxyzimtsäure-2-ethylhexylester von 77 % der Theorie, bezogen auf eingesetztes 4-Bromanisol.

### Beispiel 17

Ein Reaktionsgemisch aus 18,7 g 4-Bromanisol, 20,3 g Acrylsäure-2-ethylhexylester, 6,36 g Natriumcarbonat, 0,0025 g Palladium(II)-acetat, 0,21 g Triphenylphosphan und 2,0 g Polyethylenglykol 400 wurde unter Stickstoff auf 170°C erhitzt. Der gaschromatographisch ermittelte Umsatz an Bromanisol betrug nach 3 Stunden 97 %.

### Beispiel 18

Es wurde vorgegangen wie in Beispiel 17, jedoch wurden 1,95 g 2-Ethyl-1-hexanol zugesetzt. Der Umsatz nach 2 Stunden betrug 96 %.

### Beispiel 19

Es wurde vorgegangen wie in Beispiel 17, jedoch wurden 6,5 g 2-Ethyl-1-hexanol zugesetzt. Der Umsatz nach 3 Stunden betrug 97 %.

### Beispiel 20

Ein Reaktionsgemisch aus 18,7 g 4-Bromanisol, 20,3 g Acrylsäure-2-ethylhexylester, 6,36 g Natriumcarbonat, 0,0025 g Palladium(II)-acetat, 0,21 g Triphenylphosphan und 1,43 g 1,12-Octadecandiol wurde unter Stickstoff auf 170°C erhitzt. Der gaschromatographisch ermittelte Umsatz an Bromanisol betrug nach 3 Stunden 72 %.

### Beispiel 21

Ein Reaktionsgemisch aus 18,7 g 4-Bromanisol, 20,3 g Acrylsäure-2-ethylhexylester, 6,36 g Natriumcarbonat, 0,0025 g Palladium(II)-acetat, 0,21 g Triphenylphosphan und 5,0 g Polyethylenglykol 1000 wurde unter Stickstoff auf 170°C erhitzt. Der gaschromatographisch ermittelte Umsatz an Bromanisol betrug nach 2 Stunden 100 %. Nach Vakuumdestillation wurden 82 % Ausbeute an 4-Methoxyzimtsäure-2-ethylhexylester, bezogen auf umgesetztes 4-Bromanisol, erhalten.

### Beispiel 22

In einem Vierhalsrundkolben wurden 187,0 g 4-Bromanisol, 202,7 g Acrylsäure-2-ethylhexylester, 63,8 g Natriumcarbonat, 7,3 g 2-Ethylhexan-1,3-diol und 20 g Polyethylenglykol 400 vorgelegt und mit 2,1 g Triphenylphosphan und 0,023 g Palladium(II)-acetat versetzt (Molverhältnis Pd:Phosphor 1:80).

Das Gemisch wurde unter Stickstoff 3,5 Stunden unter kräftigem Rühren auf 160-170°C erhitzt. Nach dieser Zeit betrug der Umsatz 95 %, bezogen auf eingesetztes 4-Bromanisol.

Nach Absaugen und Destillation im Vakuum (0,1 mbar) wurde eine Ausbeute von insgesamt 88 % der Theorie (bezogen auf umgesetztes 4-Bromanisol) an 4-Methoxyzimtsäure-2-ethylhexylester isoliert.

## Patentansprüche

1. Verfahren zur Herstellung von Zimtsäurederivaten aus Bromaromaten und Acrylsäurederivaten in Gegenwart von Palladium-Katalysatoren und einem Phosphan, dadurch gekennzeichnet, daß man in Gegenwart von einer anorganischen Base, von Phosphan in großem Überschuß (bezogen auf Palladium) und von Alkoholen und/oder von einem Phasen-Transfer-Katalysator arbeitet.

2. Verfahren nach Anspruch 1, dadurch gekennzeichnet, daß man als Palladium-Katalysatoren Palladium-Verbindungen der Oxidationsstufe 0 und/oder +2 in Mengen von 0,0001 bis 1 Mol-% Palladium (bezogen auf eingesetzte Bromaromaten) einsetzt.

3. Verfahren nach Ansprüchen 1 und 2, dadurch gekennzeichnet, daß man ein Acrylsäure-Derivat der Formel (I) einsetzt 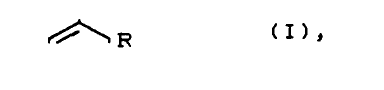 in der
R für CN oder COR¹ steht, wobei R¹ OH, O-C₆-C₁₀-Aryl, O-C₁-C₂₀-Alkyl, NH₂, NH-C₆-C₁₀-Aryl, NH-C₁-C₂₀-Alkyl, N-C₆-C₁₀-Aryl, N-C₁-C₂₀-Alkyl-C₆-C₁₀-Aryl oder N-di-C₁-C₂₀-Alkyl bedeutet.

4. Verfahren nach Ansprüchen 1 bis 3, dadurch gekennzeichnet, daß man als Bromaromaten ein Brombenzol der Formel (II) einsetzt 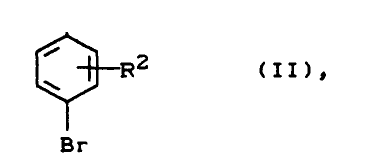 in der
R² für Wasserstoff, C₆-C₁₀-Aryl, C₁-C₂₀-Alkyl, OR³ oder NR³₂ mit R³ Wasserstoff, C₆-C₁₀-Aryl oder C₁-C ₂₀-Alkyl steht,
und ein Molverhältnis von Bromaromat zu Acrylsäure-Derivat im Bereich von 1:0,7 bis 1:3 einhält.

5. Verfahren nach Ansprüchen 1 bis 4, dadurch gekennzeichnet, daß man als anorganische Base Alkali- und/oder Erdalkalisalze schwacher Säuren in Mengen von 0,8 bis 2 Mol (pro Mol Bromaromat) einsetzt.

6. Verfahren nach Ansprüchen 1 bis 5, dadurch gekennzeichnet, daß man Kronenether, jeweils mit organischen Resten substituierte Ammonium- oder Phosphoniumsalze oder Polyethylenglykole als Phasentransfer-Katalysatoren in einer Menge von 0,01 bis 50 Mol-% (bezogen auf eingesetzten Bromaromat) einsetzt.

7. Verfahren nach Ansprüchen 1 bis 6, dadurch gekennzeichnet, daß man in Gegenwart eines oder mehrerer C₂-C₂₄-Alkohole arbeitet, wobei man in Gegenwart von Phasentransfer-Katalysatoren 3 bis 30 Mol-% dieser Alkohole (bezogen auf eingesetzte Bromaromaten) und in Abwesenheit von Phasentransfer-Katalysatoren 0,1 bis 2 l dieser Alkohole pro Mol Bromaromat einsetzt.

8. Verfahren nach Ansprüchen 1 bis 7, dadurch gekennzeichnet, daß man ein Phosphan der Formel (III) einsetzt 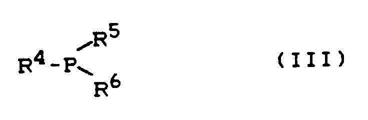 in der
R⁴, R⁵, R⁶ unabhängig voneinander jeweils für gegebenenfalls durch Halogen, C₁-C₂₀-Alkyl, OR⁷ oder NR⁷₂ (R⁷ = Wasserstoff oder C₁-C₂₀-Alkyl), substituiertes C₆-C₁₀-Aryl, C₁-C₂₀-Alkyl, Phosphino-C₁-C₂₀-Alkyl oder Phosphino-C₆-C₁₀- Aryl
stehen,
und ein Molverhältnis von Palladium:Phosphor von 1:4 bis 1:150 einhält.

9. Verfahren nach Ansprüchen 1 bis 8, dadurch gekennzeichnet, daß man es bei Temperaturen im Bereich von 100 bis 300°C durchführt.

10. Verfahren nach Ansprüchen 1 bis 9, dadurch gekennzeichnet, daß man es unter einem Schutzgas und unter Rühren durchführt.
